# EUROPEAN PATENT SPECIFICATION

(11) **EP 3 089 698 B1**
(45) Date of publication and mention of the grant of the patent: **28.08.2019**
(21) Application number: 14814900.8
(22) Date of filing: 19.12.2014
(51) Int. Cl.: A61C 11/00, A61C 9/00, A61C 13/34, G06F 17/50

(54) **METHOD AND SYSTEM FOR SCANNING TEETH MODELS**
VERFAHREN UND SYSTEM ZUM SCANNEN VON ZAHNMODELLEN
PROCÉDÉ ET SYSTÈME DE BALAYAGE DE MODÈLES DE DENT

(30) Priority: 30.12.2013 DK 201370825
(43) Date of publication of application: 09.11.2016
(73) Proprietor: 3Shape A/S, 1060 Copenhagen K (DK)
(72) Inventor: FISKER, Rune, DK-2830 Virum (DK); NONBOE, Sven, DK-3400 Hillerød (DK); HØJGAARD ALLIN, Thomas, DK-3060 Espergærde (DK); LUNDHOLDT, Rasmus, DK-2300 Copenhagen S (DK)
(74) Representative: Guardian IP Consulting I/S
(86) International application number: PCT/EP2014/078771
(87) International publication number: WO 2015/101526

(56) References cited:
- WO-A1-2011/103876
- US-A1- 2002 081 554

## Description

### Technical field

This invention generally relates to a method, an occlusion setup fixture, and a scanner system for use in recording digital 3D representations of physical teeth models. More particularly, the invention relates to a method, an occlusion setup fixture, and a scanner system for use in virtually arranging the digital 3D representations according to the patient's occlusion.

### Background

When a dental restoration is manufactured for a patient, it is important that the contact between the dental restoration and the antagonist tooth or teeth, i.e. the opposing tooth or teeth that the dental restoration engages with when the upper and lower jaw is in occlusion, is taken into account to provide an anatomical correct restoration.

When a dental restoration, such as a single tooth crown restoration or a bridge restoration, is designed and manufactured using dental CAD/CAM technology the dentist or his assistant often obtains an impression of the teeth in the patient's upper and lower jaws and produce physical teeth models, e.g. in a plaster material, from these impressions. The physical models are then scanned to obtain digital 3D representations of the teeth using e.g. a desktop scanner such as the 3shape D900 scanner. Based on the obtained digital 3D representations, a dental technician can then design the dental restoration such that it engages with the antagonist in an anatomically correct manner when the patient bites, i.e. when the teeth are brought into occlusion.

In order to arrange the digital 3D representations in a common coordinate system such that the digital 3D representations are arranged according to the patient's occlusion and to determine a digital transformation which provides this prior art methods require that a third scan is obtained in which the physical teeth models are arranged in occlusion. This third scan is often referred to as a bite scan. A bite impression may be used for aligning the physical models in occlusion but often the dental technician can determine the physical relative arrangement by mimicking normal chewing motions and placing the teeth models according to the best fit. When the upper and lower models are arranged in occlusion, with or without the use of a bite impression, the bite scan is obtained, where the bite scan comprises data relating to the surfaces of both the upper and the lower teeth models such that the bite scan can be used for virtually arranging the digital 3D representations of the teeth models in occlusion. However arranging the physical teeth models in a temporarily fixed arrangement for the recording the bite scan and the bite scan itself takes time.

Many 3D scanners have a stage or object holder on which the scanned object is arranged and the scanner operate by moving this stage relative to the optical system of the scanner or vice versa. If a scanned object is not fixed during the scanning is may be displaced during the scanning. This may cause an error in the recorded bite scan. Often the dental technician who performs the scanning procedure uses a rubber band to firmly hold the physical teeth models of the upper and lower jaws together to avoid such displacements during the bite scan. However, the use of rubber bands is evidently not a very precise method for arranging the physical teeth models and this approach may be a source of alignment error.

DE102008060504B4 describes a 3D scanner system for recording digital 3D representations of physical teeth models arranged in an articulator and a device for transforming the recorded data from a sensor coordinate system into the coordinate system of the articulator.

US 2002/0081554 A1 discloses a method to achieve bite registration of scanned images of dental study casts and the corresponding system thereof.

However, it remains a problem to provide a method and a system which can record digital 3D representations of physical teeth models and virtually arrange these according to the patient's occlusion based only on a scan of the teeth model of the upper jaw and a scan of the teeth model of the lower jaw.

### Summary

Disclosed is a method for recording digital 3D representations of physical teeth models of a patient's jaws for arranging the digital 3D representations in a common coordinate system such that the digital 3D representations are arranged according to the patient's occlusion, said method comprising:
- obtaining an occlusion setup fixture comprising a first and a second model interface each configured for supporting a teeth model and a guiding structure configured for providing a confined relative movement of the first and second model interfaces in the fixture;
- obtaining a first teeth model and attaching it to said first model interface such that a first assembly comprising the first teeth model and at least part the first model interface is formed;
- obtaining a second teeth model and arranging it in occlusion with the first teeth model;
- attaching the second model interface to the second teeth model while the first and second teeth models are kept in occlusion such that a second assembly comprising the second teeth model and at least part of the second model interface is formed, where the second model interface and the second teeth model are brought into contact by the confined relative movement of the model interfaces;
- placing the first and second assemblies in a 3D scanner and recording a first and a second digital 3D representation of the first and second teeth models, respectively, where the relative arrangement of the model interface parts of the assemblies within the coordinate system of the 3D scanner is detected or predetermined;
- obtaining information describing the confined relative movement of the model interfaces in the occlusion setup fixture; and
- virtually bringing the recorded digital 3D representations into occlusion by a virtual movement, where the virtual movement comprises a virtual translation along a path according to the confined relative movement.

Disclosed is a scanner system for recording digital 3D representations of physical teeth models of a patient's jaws and arranging the digital 3D representations in a common coordinate system such that the digital 3D representations are arranged according to the patient's occlusion, said system comprising:
- an occlusion setup fixture comprising a first and a second model interface each configured for supporting a teeth model, and a guiding structure configured for providing a confined relative movement of the first and second model interfaces in the fixture;
- a 3D scanner configured for recording digital 3D representations of the teeth models; and
- a data processing unit comprising a non-transistory computer readable medium on which is stored computer implemented algorithms for virtually bringing the recorded digital 3D representations into occlusion by a virtual movement, where the virtual movement comprises a translation along a path according to the confined relative movement.

Disclosed is an occlusion setup fixture for transferring the relative arrangement of physical teeth models in occlusion to the coordinate system of a 3D scanner, wherein the fixture comprises:
- a first model interface and a second model interface, where each model interface is configured for supporting a teeth model; and
- a guiding structure comprising one or more elongated posts defining a linear axis of the fixture;
where the first and second model interfaces are configured for directly or indirectly engaging said one or more elongated posts such that the relative movement of the first and second model interfaces is confined to a linear movement along the linear axis.

The confined relative movement of the model interfaces in the fixture provides that the relative position of the teeth model and model interface in the second assembly depends on the occlusion and the relative position of the teeth model and model interface in the first assembly.

The arrangement of the first teeth model on the first model interface can be determined by the operator when he fixates the teeth model to the model engaging surface of the model interface using e.g. an adhesive material. When the first model is fixated it preferably remains in the same position on the first model interface while used in the occlusion setup fixture and during the scanning of the first teeth model in the 3D scanner. The same is true for the second physical teeth model and the second model interface where the arrangement of the second teeth model on the second model interface as defined in the occlusion setup fixture is maintained during the scanning of the second teeth model in the 3D scanner.

The position of a physical teeth model in the 3D scanner coordinate system is then determined by the position of the teeth model relative to the corresponding model interface and the position of this model interface relative to the coordinate system of the 3D scanner.

In some cases, the model interfaces are arranged in the 3D scanner in a predetermined position and orientation. This can be obtained by placing the assemblies in a fixed mounting part of the 3D scanner, where the mounting part and the model interfaces are configured for engaging with each other in a manner such that the model interface and hence the assemblies are arranged according to a predetermined position and orientation relative to the fixated mounting part and hence relative to the 3D scanner coordinate system.

Since the position of the second teeth model on the second model interface is determined by the position of the first teeth model on the first model interface a predetermined arrangement of the model interfaces in the 3D scanner provides that the arrangement of the second teeth model in the 3D scanner coordinate system is determined by the arrangement of the first teeth model in this coordinate system and by the patient's occlusion. I.e. the relative arrangement of the first and second teeth models in the coordinate system of the 3D scanner is determined by the patient's occlusion.

One advantage of the occlusion setup fixture is hence that the position of the second teeth model in the coordinate system of the 3D scanner is determined by the patient's occlusion and the position of the first teeth model in the 3D scanner coordinate system.

This provides for a correlation between the digital 3D representations of the first and second teeth models expressing the patient's occlusion and based on this correlation it is possible to determine the transformation which brings the two digital 3D representations into the same coordinate system with the digital 3D representations arranged according to the patient's occlusion.

The disclosed method, fixture and system thus provides for recording digital 3D representations of physical teeth models of a patient's upper and lower jaws and their relative arrangement in occlusion.

Further they provide for transferring the occlusion of the physical teeth models to the recorded digital 3D representations such that the digital 3D representations can be represented in a common coordinate system with the digital 3D representations arranged according to the patient's occlusion.

Accordingly, the proposed method, fixture and system provide several advantages over approaches relying on a bite-scan. Firstly, a two-scan procedure for recording digital 3D representation of the teeth models and determining the transformation which brings these into the same coordinate system can be realized. This evidently provides for a reduced scan time and a faster procedure. Secondly, the precision is improved since the abovementioned error in the arrangement of the physical teeth models during a bite scan is avoided.

In the context of the present disclosure, the phrase "confined relative movement" refers to the situation where the movement of a teeth model/model interface is constrained to a predetermined path by the occlusion setup fixture.

In the context of the present disclosure, the phrase "according to the patient's occlusion" refers to the situation where the physical teeth models or the digital 3D representations of these are arranged according to the anatomical correct arrangement of the corresponding teeth in the patient's mouth mimicking the relative arrangement of the jaws/teeth when the patient's mouth is closed and the jaws are stationary. In the field of dentistry this situation is often referred to as static occlusion.

The confined relative movement of the first and second model interfaces in the occlusion setup fixture provides that first and second model interfaces at all positions along the guiding structure have a known orientation and offset relative to the path of the confined relative movement.

It is advantageous that the virtual movement bringing the recorded digital 3D representations into occlusion comprises a translation along the path according to the confined relative movement of the occlusion setup fixture since this provides that the bite scan of prior art methods can be omitted. There is then no need for the dental technician to spend time on inserting the physical teeth models of the upper and lower jaws together into the scanner for the bite scan and a faster overall scan procedure can be realized.

When the bite scan is omitted, the scanning procedure is faster and requires less operator and scanner time for each set of upper and lower physical teeth models. Further the determined alignment of the upper and lower may be more precise than was can be obtained using a bite scan in which the relative arrangement of the upper and lower teeth models may be imprecise.

It is an advantage of the method and the system that the occlusion setup fixture confines the relative movement of the model interfaces. It provides that the arrangement of the second physical teeth model on the second model interface is dictated by the arrangement of the first teeth model on the model interface and by the arrangement of the first and second physical teeth models in occlusion. When the arrangement of the assemblies in the 3D scanner is known, all the information required to virtually arrange the digital 3D representations of the first and second physical teeth models according to the patient's occlusion is available.

The placement of the model interfaces in the 3D scanner may be limited to a predetermined position and orientation relative to the coordinate system of the 3D scanner. This can e.g. realized when the 3D scanner comprises a fixed mounting part which comprises structures and/or is shaped such that the model interfaces only can be arranged in one position and orientation on the mounting part. The only variable parameter in the arrangement of a teeth model in the coordinate system of the 3D scanner is then the placement of the teeth model on the model interface. The model interface may also comprise one or more structures which can be identified in a scan and used for deriving the arrangement of the model interface in the coordinate system of the 3D scanner.

The order of the step of recording a digital 3D representation of the first physical teeth model and the steps of arranging the first assembly in the occlusion setup fixture and arranging the second physical model in occlusion with the first physical teeth model is not important

When the first assembly is arranged in the occlusion setup fixture in a known arrangement, the second physical teeth model can be arranged in occlusion with the first physical teeth model. The second physical teeth model is then attached to the second model interface to form the second assembly. The first and second assemblies can then be removed from the occlusion setup fixture and arranged in the 3D scanner for recoding of the digital 3D representation. The physical teeth model of the first assembly can also be scanned before the first assembly is arranged in the occlusion setup fixture.

In some embodiments, the fixture comprises means for providing that the confined relative movement comprises a translation along a linear axis. These means may comprise at least one elongated structure, such as one or more elongated posts. At least one of said platforms or at least one of said model interfaces are then preferably configured for engaging and sliding along this elongated structure, whereby the guiding structure provides that the confined relative movement comprises a linear movement of the model interfaces along a linear axis of the fixture.

In some embodiments, the confined relative movement comprises a translation along a linear axis of the fixture

In the context of the present disclosure, the phrase "linear movement along a linear axis" refers to movement that is at least parallel to the linear axis.

In some embodiments, the fixture comprises an articulator and the confined relative movement of the model interfaces mimics the patient's bite.

The confined relative movement may then be dictated by the settings of the articulator.

In some embodiments, the virtual movement comprises a virtual 180 degree rotation of one of said digital 3D representations.

The 180 rotation can be around a rotation axis which is parallel to the patient's occlusal and medial planes. Such a rotation is advantageous when the first and second assemblies have a similar arrangement in the 3D scanner while the digital 3D representations are recorded, such as when the model interface parts of the first and second assemblies mate with the same mounting part in a sequential scanning procedure where one physical model is scanned before the other. The rotation then provides that the teeth parts of the digital 3D representations of the patient's upper and lower jaws are arranged with the teeth facing each other. The determined digital transformation then also express the 180 degrees rotation.

In some embodiments, at least one of said model interfaces comprises a platform configured for engaging the guiding structure.

In some embodiments, the model interface comprises an interface plate configured for engaging with said platform, where the platform comprises one or more positioning structures for positioning the interface plate in a predetermined position and orientation on the platform.

In some embodiments, the occlusion setup fixture comprises a first platform configured for engaging said guiding structure and the first model interface comprises a first interface plate configured for mating with the first platform.

In some embodiments the method comprises attaching the first teeth model and the first interface plate to the first platform such that the first assembly is attached to the first platform. The first assembly then preferably comprises the first teeth model and the first interface plate.

In some embodiments, the method comprises:
- releasably attaching the first assembly to the first platform with the first interface plate arranged in a known position and orientation on the first platform; or
- releasably attaching the first interface plate to the first platform in a known position and orientation and subsequently attaching the first physical teeth model to the first interface plate to form the first assembly.

In some embodiments, the first interface plate and the first platform comprises mating structures configured for providing that the first interface plate can be arranged in a pre-determined and known orientation and position on the first platform.

The mating structures may comprise mechanical fixation means such as a clip-bar and an indentation in the platform surface shaped to match the shape of the interface plate
The mating structures may comprise magnetic fixation means such as magnetic balls partly integrated in the platform and/or interface plate. Preferably, the mating means are capable of holding the interface plate in place during movement of the platforms.

The first interface plate preferably comprises a surface shaped to provide that a physical teeth model can be attached thereto such that a first assembly comprising the first interface plate and the first physical teeth model can be formed. The first physical teeth model can hence be attached to the first platform via said first interface plate, and the movement of the first physical teeth model in the occlusion setup fixture is confined by the confined movement of the platform along the guiding structure.

The first platform and the first interface plate are configured to provide that the first interface plate can be releasably fixated in a known arrangement on the first platform such that the orientation and position of the first interface plate in a plane, such as in a plane perpendicular to a linear axis of the occlusion setup fixture, is known.

In some embodiments, the occlusion setup fixture comprises a second platform configured for engaging said guiding structure and the second model interface comprises a second interface plate configured for mating with the second platform. Any movement of the second platform in the fixture is preferably confined by the guiding structure.

In some embodiments, the method comprises arranging the second interface plate at the second platform in a before attaching the second teeth model is to the second interface plate. The second assembly formed thereby thus comprises the second teeth model and the second interface plate.

This has the advantage that when the second assembly comprising the second interface plate and the second physical teeth model is formed the relative arrangement of the first and second interface plates is known from knowledge of the design of the occlusion setup fixture while the relative arrangement of the first and second physical teeth models is determined by the patient's occlusion. This provides that the transformation comprising a virtual translation mimicking the confined physical translation of the platforms/interface plates in the occlusion setup fixture.

In some embodiments, the first interface plate is configured for mating with the first platform in a predetermined orientation and position.

In some embodiments, the second interface plate is configured for mating with the second platform in a predetermined orientation and position.

When a platform engages the guiding structure with a predetermined orientation and position and the corresponding interface plate is arranged in a predetermined orientation and position on the platform, the confined movement of the interface plate in the fixture will be well-defined.

Preferably the first and second interface plates each comprise a surface shaped to provide that a physical teeth model can be attached thereto such that the first and second assemblies each comprising an interface plate and a physical teeth model can be formed.

It is advantageous that the platforms are configured to provide the attached interface plates are arranged in predetermined and known orientations relative to each other and to the linear axis of the occlusion setup fixture.

When the model interfaces comprises interface plates which engage platforms of the occlusion setup fixture, a movement of the second model interface and/or of the first assembly and the second physical teeth model according to the confined relative movement comprises confining the relative movement of the platforms and the attached assemblies suing the guiding structure until the second model interface and the second physical teeth model are brought into contact. Since each platform is configured for engaging said guiding structure the relative movement of the first and second platforms is confined by the guiding structure, and accordingly the relative movement of the first and second interface plates is also confined.

In some embodiments, the one or more elongated posts are secured in one platform and the other platform is capable of sliding along the one or more elongated posts.

In some embodiments, the first and/or second model interface comprises a first and/or second a second interface plate configured for engaging with a corresponding first and/or second platform, respectively, where the platforms are configured for engaging the guiding structure. The platforms thus act as connecting components providing that the interface plates indirectly engage the guiding structure. One advantage of such a design is that the platforms may be relatively large to provide a good coupling to the guiding structures while the interface plates can be relatively small to prevent them from taking up much of the scan volume of the 3D scanner.

In some embodiments, the first and/or second platforms comprise one or more positioning structures for positioning the interface plates in a predetermined arrangement on the platforms.

In some embodiments, the 3D scanner comprises a mounting part configured for mating with the model interfaces in such a manner that the position and orientation of the model interfaces in the 3D scanner coordinate system are known.

In some embodiments, the 3D scanner comprises a mounting part configured for mating with at least parts of the model interfaces, such as interface plates of the model interfaces, in such a manner that the parts of the model interfaces are arranged according to a predetermined position and orientation on the mounting part. If the mounting part is arranged in a predetermined position and orientation in the 3D scanner, such as in a fixed position and orientation, the position and orientation of the parts of the model interfaces in the 3D scanner coordinate system are known.

In some embodiments, the 3D scanner comprises a mounting part configured for mating with a model interface in such a manner that the first and second assemblies can be arranged in the 3D scanner in a known and predetermined position and orientation.

In some embodiments, the method comprises arranging the first and second assemblies in a known and predetermined position and orientation in a mounting part of the 3D scanner.

In some embodiments, the interface plate is adapted for engaging a mounting part of a 3D scanner in manner such that when arranged on the mounting part the interface plate has a predetermined position and orientation in the coordinate system of the 3D scanner.

In some embodiments, the interface plate comprises one or more structures adapted for engaging corresponding structures on a fixed mounting part of a 3D scanner, such that when arranged in the mounting part the interface plates have a predetermined position and orientation in the coordinate system of the 3D scanner.

Alternatively or in addition to such structures, the interface plates may be shaped such that they have a reduced rotation symmetry around a normal to the surface configured for supporting a teeth model. If the mounting part then has e.g. a recess shaped according to the shape of the interface plates, the recess provides that the interface plates will also have a predetermined position and orientation in the coordinate system of the 3D scanner.

Both approaches can provide a predetermined arrangement of the first and/or second model interfaces within the coordinate system of the 3D scanner.

In some embodiments, the method comprises arranging the first and/or second assembly in an interface plate mount of the 3D scanner where said interface plate mount is configured for mating with the interface plates in a known manner such that the position and orientation of the interface plates in the scanner coordinate system are known. The known position and orientation may be a predetermined position and orientation determined by the design of the interface plate mount and the interface plates.

It is an advantage that the interface plates, the platforms and the mounting part of the 3D scanner are configured to provide that the interface plates have a known arrangement in both the occlusion setup fixture and the 3D scanner. In such cases the digital 3D representations of the physical teeth models can be virtually arranged according to the patient's occlusion by said transformation.

In some embodiments, the step of placing the first and second assemblies in the 3D scanner comprises arranging the assemblies with the model interfaces engaging the mounting parts.

In some embodiments, the first and second assemblies are arranged one at a time in the same position and orientation relative to the coordinate system if the 3D scanner. This has the advantage that the transformation only includes a rotation around said rotation axis and the virtual translation along the linear axis.

In some embodiments, the mounting part of the 3D scanner and the interface plates are configured to provide that the interface plates only can be arranged in one predetermined and known orientation and position relative to the coordinate system of the 3D scanner.

In some embodiments, the first and/or second model interfaces are configured for engaging said guiding structure. In such cases there is no need for a platform to provide the confined movement of the model interface within the occlusion setup fixture.

In some embodiments, at least one of said model interfaces comprises one or more structures or a shape which can be identified in a digital 3D representation and used for determining the position and orientation of the model interface in the 3D scanner.

This can be realized using e.g. CAD models of said structures are loaded into the data processing unit and aligned with the corresponding parts of the digital 3D representation.

In some embodiments, the method comprises performing a calibration scan to determine the position and orientation of the model engaging surface of the model interface in the coordinate system of the 3D scanner, and where the virtual movement comprises the rotation and/or movement required to compensate for a misalignment of the model engaging surface relative to the 3D scanner coordinate system.

Such as misalignment can e.g. relate to the normal of the model engaging surface is not aligned with the z-axis of the 3D scanner coordinate system such that the model engaging surface is tilted relative to the x-y plane of the coordinate system. The misalignment may also be that the model interface is rotated around its normal such that its direction is offset relative to the x and y axes of the 3D scanner coordinate system. Also the center of the model interface may be offset from the expected position.

The calibration scan may record a digital 3D representation of part of the model interface, such as part of an interface plate, only when this is mounted in the mounting part of the 3D scanner. If the model interface comprises structures and/or has a shape allowing the interface plate to be identified in the digital 3D representation this can be used to determine e.g. the normal and center of the model interface. When the model interface has the shape of a truncated circle, the circular portion can be used to identify the center, while the straight section in the truncated part can be used to determine the direction of the model interface and its rotation around the normal vector.

The virtual movement required to compensate for the misalignment may be applied to both the first and second digital 3D representations.

In some embodiments, the method comprises performing a calibration scan of a calibration object to determine the angular offset between the linear axis of the guiding system and the normal vectors of the model interfaces when arranged in the occlusion setup fixture. In some embodiments, the transformation is configured to compensate for the determined angular offset.

Often the teeth models are scanned one at a time with the teeth models and their interfaces arranged in the same position in the 3D scanner. The obtained digital 3D representations are thus overlapping in the coordinate system of the 3D scanner.

In some embodiments, the method thus comprises a separation of the digital 3D representations taking at least one of these along the path according to the confined relative movement until the digital 3D representations no longer overlap.

In some embodiments, a collision detection between the first and second digital 3D representations is used to determine when to stop the virtual translation. The collision detection is preferably performed when the 180 degree rotation has been applied to one of the two digital 3D representations. When the digital 3D representations are separated at the onset of the virtual translation, the virtual translation preferably continues until there is an overlap between the digital 3D representations, i.e. until there is a collision between the two.

When the digital 3D representations overlap at the onset of the virtual translation the virtual translation preferably continues until the overlap between the digital 3D representations is below a predefined limit or to the point where there is just no collision.

In some embodiments, the method comprises an occlusion optimization where an optimized relative arrangement of the first and second digital 3D representations is detected and the virtual movement comprises a move to the detected optimized relative arrangement.

The optimization may be based on an optimization of the area of contact between the digital 3D representations.

In such cases the digital transformation also express the virtual movement taking the taking the digital 3D representations to the adjusted relative arrangement.

In some embodiments, the arrangement of the interface parts of the first and/or second assembly within the coordinate system of the 3D scanner is detected from structures of the model interface identified in the first and/or second digital 3D representations, respectively. The structures may be formed as separate elements having easily detectable shapes such as cylindrical protrusions where CAD models of these protrusions can be aligned with corresponding parts of the digital 3D representation to determine their positon in the digital 3D representation.

This approach provides the advantage that there is no requirement for arranging the first and second assemblies in the same position and orientation in the 3D scanner.

In some embodiments, the occlusion setup fixture is configured to provide that at least one of the platforms can be removed from the linear guiding structure and the platform is capable of fitting into the mounting portion of the 3D scanner.

In some embodiments at least one platform is configured for direct attachment of the teeth model and comprises one or more structures adapted for engaging corresponding structures on a mounting part of a 3D scanner, such that when arranged at the mounting part the platform has a predetermined position and orientation in the coordinate system of the 3D scanner.

The platforms thus acts as the model interfaces. This provides the advantage that a device with a low number of components can be realized and the one source of inaccuracy is eliminated.

In some embodiments, the first physical teeth model is equipped with a base configured for engaging a base accepting portion of the first platform where the base and the base accepting portion are configured to provide that the first physical teeth model is arranged in a predetermined and known arrangement relative to the first platform. The mounting part of the 3D scanner is then preferably shaped such that the physical teeth model only can be arranged in a predetermined and known orientation and position in the coordinate system of the 3D scanner.

The present disclosure relates to different aspects including the method and system described above and in the following, and corresponding methods and systems each yielding one or more of the benefits and advantages described in connection with the first mentioned aspect, and each having one or more embodiments corresponding to the embodiments described in connection with the first mentioned aspect and/or disclosed in the appended claims.

Disclosed is a method for recording digital 3D representations of physical teeth models of a patient's jaws and for arranging the digital 3D representations according to the patient's occlusion, said method comprising:
- obtaining an occlusion setup fixture comprising a first and a second model interface each configured for supporting a physical teeth model and a linear guiding structure defining a linear axis, where the occlusion setup fixture is configured to provide that the relative movement of the first and second model interfaces is confined to translation along the linear axis;
- obtaining a first physical teeth model and attaching it to said first model interface such that a first assembly comprising the first model interface and the first physical teeth model is formed;
- obtaining a second physical teeth model and arranging it in occlusion with the first physical teeth model;
- attaching the second model interface to the second physical teeth model while the first and second physical teeth models are kept in occlusion such that a second assembly comprising the second model interface and the second physical teeth model is formed, where the second model interface and the second physical teeth model are brought into contact by a translation of the second model interface and/or of the first assembly and the second physical teeth model along the linear axis;
- placing the first and second assemblies in a 3D scanner with the model interfaces arranged according to a predetermined location and orientation within the 3D scanner, and recording digital 3D representations of the first and second physical teeth models; and
- virtually bringing the recorded digital 3D representations into occlusion by a transformation comprising a virtual translation along the linear axis of the occlusion setup fixture.

Disclosed is a scanner system for recording digital 3D representations of physical teeth models of a patient's jaws and for arranging the digital 3D representations according to the patient's occlusion, said system comprising:
- an occlusion setup fixture comprising a first and a second model interface each configured for supporting a physical teeth model and a linear guiding structure defining a linear axis, where the occlusion setup fixture is configured to confine the relative movement of the first and second model interfaces to translations along the linear axis;
- a 3D scanner configured for recording digital 3D representations of physical teeth models supported by the model interfaces; and
- a data processing unit comprising a non-transistory computer readable medium on which is stored computer implemented algorithms for virtually bringing recorded digital 3D representations of physical teeth models into occlusion by a transformation comprising a virtual translation along the linear axis of the occlusion setup fixture.

In particular, disclosed herein is a method for recording digital 3D representations of physical teeth models of a patient's upper and lower jaws and for arranging the digital 3D representations according to the patient's occlusion, said method comprising:
- obtaining an occlusion setup fixture comprising a first and a second platform and a linear guiding structure defining a known linear axis, where each platform is configured for engaging said guiding structure such that the relative movement of the first and second platforms is confined by the guiding structure to translations along the known linear axis, the occlusion setup fixture further comprising a first and a second interface plate each releasably attached in predetermined arrangements to the first and second platform, respectively;
- obtaining a first physical teeth model and attaching it to said first platform via said first interface plate such that a first assembly comprising the first interface plate and the first physical teeth model is formed;
- obtaining a second physical teeth model and arranging it in occlusion with the first physical teeth model;
- translating the second platform and/or the first platform and the physical teeth models along the guiding structure until the second platform contacts the second physical teeth model and attaching the second platform to the second physical teeth model while the first and second physical teeth models are kept in occlusion, where the second physical model is attached to the second platform via said second interface plate such that a second assembly comprising the second interface plate and the second physical teeth model is formed;
- recording digital 3D representations of the first and second physical teeth models by scanning the first and second physical teeth models separately in a 3D scanner while the first and second assemblies are arranged in an interface plate mount of the 3D scanner, where the interface plate mount is configured for mating with the first and second interface plates in a pre-determined manner such that the position and orientation of the interface plates in the 3D scanner coordinate system are known; and
- virtually bringing the recorded digital 3D representations into occlusion by a transformation comprising a virtual translation along the known linear axis of the occlusion setup fixture.

Disclosed is a method for arranging digital 3D representations of physical teeth models in a common coordinate system such that the digital 3D representations are arranged according to the patient's occlusion, said method comprising:
- obtaining a first digital 3D representation of a first teeth model of one of the patient's jaws;
- obtaining a second digital 3D representation of a second teeth model of the opposing jaw;
   where the first and second teeth models are arranged on first and second model interfaces of an occlusion setup fixture, respectively, while the digital 3D representations are obtained, and the position and orientation of the second teeth model on the second model interface is determined by the patient's occlusion and the position and orientation of the first teeth model on the first model interface; and
- obtaining information describing a confined relative movement of the model interfaces in the fixture.

Disclosed is a method for recording digital 3D representations of physical teeth models of a patient's jaws and determining a digital transformation for arranging the digital 3D representations in a common coordinate system such that the digital 3D representations are arranged according to the patient's occlusion, said method comprising:
- bringing the recorded digital 3D representations into occlusion in a common coordinate system using one of the disclosed embodiments; and
- determining the digital transformation from the virtual movements used to bring the digital 3D representations into occlusion.

The digital transformation preferably expresses the virtual movement.

Disclosed is a system for recording digital 3D representations of physical teeth models of a patient's jaws and determining a digital transformation for arranging the digital 3D representations in a common coordinate system such that the digital 3D representations are arranged according to the patient's occlusion, said system comprising a data processing unit comprising a non-transistory computer readable medium on which is stored computer implemented algorithms for:
- bringing the recorded digital 3D representations into occlusion in a common coordinate system using one of the disclosed embodiments; and
- determining the digital transformation from the virtual movements used to bring the digital 3D representations into occlusion.

Furthermore, the disclosure relates to a computer program product comprising program code means for causing a data processing system to perform the virtual translation according to any of the embodiments, when said program code means are executed on the data processing system, and a computer program product, comprising a computer-readable medium having stored there on the program code means.

### Brief description of the drawings

The above and/or additional objects, features and advantages of the present invention, will be further elucidated by the following illustrative and nonlimiting detailed description of embodiments of the present invention, with reference to the appended drawings, wherein:
Fig. 1 shows flowcharts for embodiments of the method
Figs. 2 shows an embodiment of an occlusion setup fixture.
Fig. 3 shows a schematic of a system according to an embodiment.
Fig. 4 shows a schematic of a method according to an embodiment.

### Detailed description

In the following description, reference is made to the accompanying figures, which show by way of illustration how the invention may be practiced.

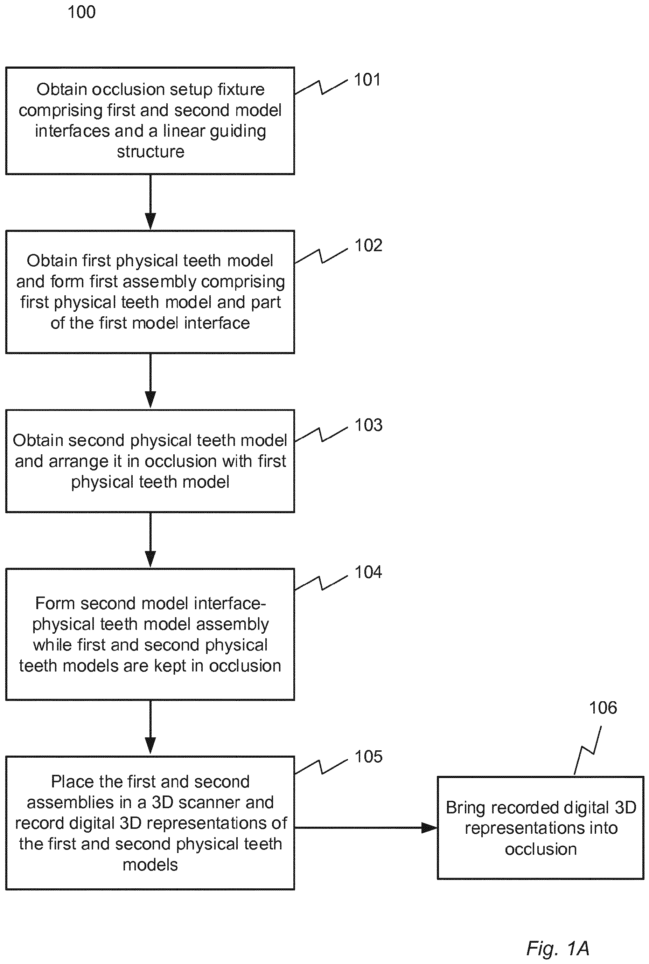

Fig. 1A shows a flowchart 100 of an embodiment of the method for recording digital 3D representations of physical teeth models of a patient's jaws and for determining the digital transformation for arranging the digital 3D representations in a common coordinate system such that the digital 3D representations are arranged according to the patient's occlusion.

In step 101 an occlusion setup fixture is obtained. The occlusion setup fixture comprises a first and a second model interface each configured for supporting a teeth model and a linear guiding structure defining a linear axis. The occlusion setup fixture is configured to provide that when the model interfaces are arranged in the occlusion setup fixture the relative movement of the first and second model interfaces within the occlusion setup fixture is confined to translation along the linear axis defined by the guiding structure. The relative movement of teeth models attached to the model interfaces is then also confined to this linear translation.

A first teeth model is obtained in step 102 and attached to the first model interface such that a first assembly comprising the first teeth model and at least part of the first model interface is formed. The first teeth model can be attached to the first model supporting element before or after the model supporting element is arranged in the occlusion setup fixture.

A second teeth model is obtained and arranged in occlusion with the first teeth model in step 103 according to a best fit evaluated by the user. For teeth models representing a large part of the patient's set of teeth, the upper teeth model can simply rests on the lower teeth model when these are arranged in occlusion.

In step 104, the second model interface is attached to the second teeth model while the first and second teeth models are kept in occlusion. The second model interface and the second teeth model are brought into contact by a translation of the second model interface and/or of the first assembly and the second teeth model along the linear axis. In some cases it is most convenient to move the second model interface along the linear axis until it contacts the second teeth model, while in other cases the occlusion setup fixture is designed such that the second teeth model, the first teeth model and the first model interface are moved towards the second model interface along the linear axis. Regardless of which part or parts which are moved towards the other, a second assembly comprising the second teeth model and the second interface plate is formed, where the orientation and position of the second teeth model on the second model interface is determined from the orientation and position of the first teeth model on the first model interface, and the patient's occlusion. The arrangement of the second teeth model relative to the second model interface is thus dictated by the relative arrangement of the first and second teeth models in occlusion.

In step 105, the first and second assemblies are placed in a 3D scanner with the model interfaces arranged according to a predetermined location and orientation within the 3D scanner, i.e. the position and orientation of the model interfaces in the scanner coordinate system are known, and digital 3D representations of the first and second teeth models are recorded. This predetermined arrangement of the model interfaces within the 3D scanner can be achieved using a 3D scanner comprising a fixated mounting part configured for mating with the model interfaces in a unique arrangement. The digital 3D representations of the first and second teeth models are then recorded by scanning the first and second teeth models separately in the 3D scanner, either simultaneously or one at a time.

In step 106 the recorded digital 3D representations are virtually brought into occlusion by a transformation comprising a virtual translation along the linear axis of the occlusion setup fixture.

In some cases the model interfaces themselves engage the guiding structure of the occlusion setup fixture, such that the model interfaces both acts as the platform which provides that the guiding structure can confine the relative movement of the model interfaces and as the part which engages a part of the 3D scanner to provide a predetermined orientation and position.

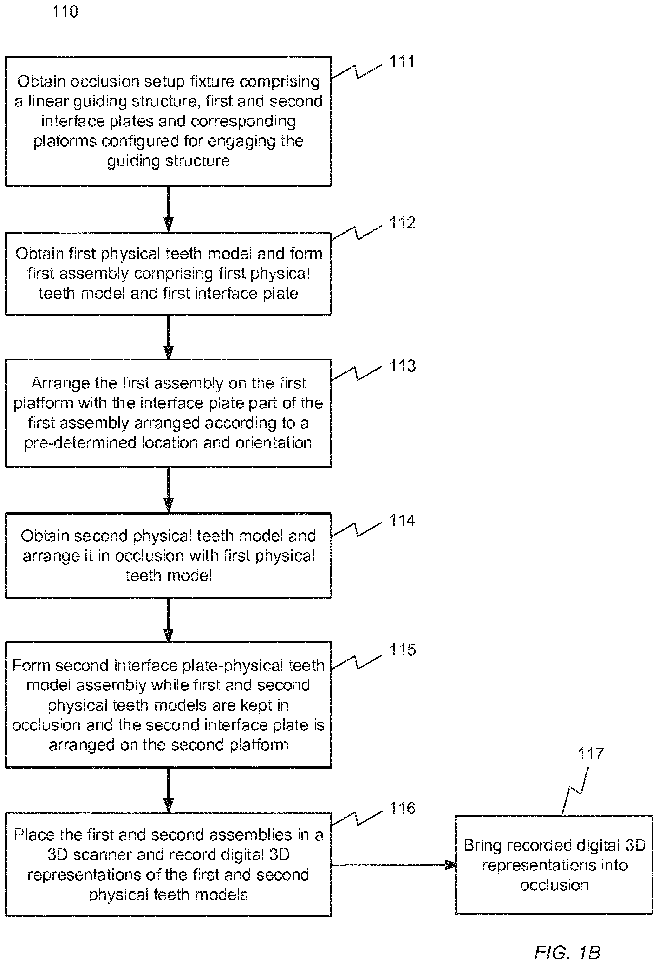

In other cases, as the embodiment described below in relation to Fig. 1B, the occlusion setup fixture comprises an interface plate which engages a platform of the occlusion setup fixtures and a mounting part of the 3D scanner.

Fig. 1B shows a flowchart 110 of an embodiment of the method for recording digital 3D representations of physical teeth models of a patient's jaws and for arranging the digital 3D representations in a common coordinate system such that the digital 3D representations are arranged according to the patient's occlusion, where the relative arrangement of the first and second teeth models in occlusion is transferred to the 3D scanner coordinate system using interface plates.

In step 111 an occlusion setup fixture is obtained. The occlusion setup fixture comprises a first and a second platform and a linear guiding structure defining a linear axis occlusion setup fixture, where each platform is configured for engaging the guiding structure such that the relative movement of the first and second platforms is confined by the guiding structure to translations along the linear axis. The occlusion setup fixture further comprises first and second interface plates acting as model interfaces and supporting the teeth models both in the occlusion setup fixture and in the 3D scanner. The interface plates are configured to be releasably attached to the first and second platform in a known orientation and position.

In step 112 a first teeth model is obtained and attached to the first interface plate to form a first interface plate- teeth model assembly. The first assembly is arranged is arranged on the first platform in step 113 with the interface plate part of the first assembly engaging the platform such that first teeth model is attached to the first platform via said first interface plate.

A second teeth model is obtained and arranged in occlusion with first teeth model in step 114.

In step 115 the second interface plate is releasably attached to the second platform with the second interface plate arranged according to a known orientation and position on the second platform. The second platform with the second interface plate and/or the first platform with the teeth models and the first interface plate are then translated along the guiding structure until the second interface plate contacts the second teeth model. The second interface plate is then attached to the second teeth model such that a second interface plate- teeth model assembly is formed. The first and second teeth models are kept in occlusion during the translation the first and second teeth models are kept in occlusion and the forming of the second assembly, such that the relative arrangement of the second teeth model and the second interface plate is dictated by the relative arrangement of the first teeth model and the first interface plate, and the arrangement of the first and second teeth models in occlusion.

In step 116, the first and second assemblies are placed in a 3D scanner with the interface plates arranged according to a predetermined location and orientation within the 3D scanner, i.e. the position and orientation of the interface plates in the scanner coordinate system are known, and digital 3D representations of the first and second teeth models are recorded. The predetermined location and orientation within the 3D scanner can be achieved by mounting the assemblies in a mounting part of the 3D scanner which is configured for mating with the interface plates in such a manner that the position and orientation of the model contacting parts in the 3D scanner coordinate system are known. This predetermined arrangement of the model interfaces within the 3D scanner can be achieved using a 3D scanner comprising a mounting part configured for mating with the interface plates in a unique and known arrangement. The digital 3D representations of the first and second teeth models are preferably recorded by scanning the first and second teeth models separately in the 3D scanner, either simultaneously or one at a time.

In step 117 the recorded digital 3D representations are virtually brought into occlusion by a transformation comprising a virtual translation along the linear axis of the occlusion setup fixture.

The arrangement of the second teeth model on the second interface plate is determined by the relative arrangement of the first and second teeth models in occlusion and by the linear guiding structure of the occlusion setup fixture which confines the relative movement of the first and second platforms to translations along the known linear axis of the occlusion setup fixture. Once the first I teeth model is arranged on the first interface plate, there is only one possible arrangement of the second teeth model on the second interface plate and this possible arrangement is characterized by the occlusion of the patient's teeth.

The digital 3D representations of the first and second teeth models recorded by the 3D scanner describes the surface of the first and second teeth model in the coordinates of the 3D scanner. The mounting part of the 3D scanner and the interface plates are configured to provide that the interface plates can only be arranged in a predetermined orientation and position relative to the coordinate system of the 3D scanner.

The position and orientation of the digital 3D representations of the first and second physical teeth models in the coordinate system of the of the 3D scanner is hence locked to each other due to the confined movement of the first and second platforms in the occlusion setup fixture and the predetermined arrangement of the first and second interface plates in the coordinate system of the of the 3D scanner.

The digital transformation bringing the recorded digital 3D representation of the first and second teeth models into occlusion comprises a virtual translation along the known linear axis of the occlusion setup fixture. When the mounting part of the 3D scanner is configured such that the first and second assemblies are arranged in the same position and orientation in the 3D scanner, the virtual transformation preferably also comprises a rotation providing a 180 degrees rotation to one of the digital 3D representations bringing the digital 3D representation of one of the teeth models into a position relative to the other which mimics the relative arrangements of the teeth models in the occlusion setup fixture and thus mimics the relative arrangement of the patient's teeth in occlusion.

A coordinate system of the occlusion setup fixture can be defined such that the z-axis is aligned with the linear axis defined by the linear guiding structure. The surface of the first teeth model is described by a set of coordinates (x1, y1, z1) in the coordinate system of the occlusion setup fixture. In the same coordinate system the surface of the second teeth model is described by a set of coordinates (x2, y2, z2) given by the relative arrangement of the teeth models in occlusion and (x1, y1, z1).

The relative arrangement of the physical teeth models in occlusion is hence transferred to the coordinate system of the interface plates using the occlusion setup fixture. The exact arrangement of the individual teeth model on their respective interface plate is unknown, but since the relative arrangement of the first and second teeth models in their in occlusion is unique and the first and second interface plates are locked in their relative movement to a translation along the known axis of the occlusion setup fixture, the relative arrangement of the first and second teeth models in occlusion can be transferred to the coordinate system of the 3D scanner by arranging the interface plates (with the teeth models) in predetermined locations and orientations in the 3D scanner, such as by arranging the assemblies with the interface plates mating with the mounting part of the 3D scanner.

The digital 3D representations of the first and second teeth models are recorded in the coordinate system of the 3D scanner. The arrangement of each teeth model in the scanner coordinate system is given by the arrangement of the teeth model on the interface plate and the arrangement of the interface plate on the mounting part of the scanner.

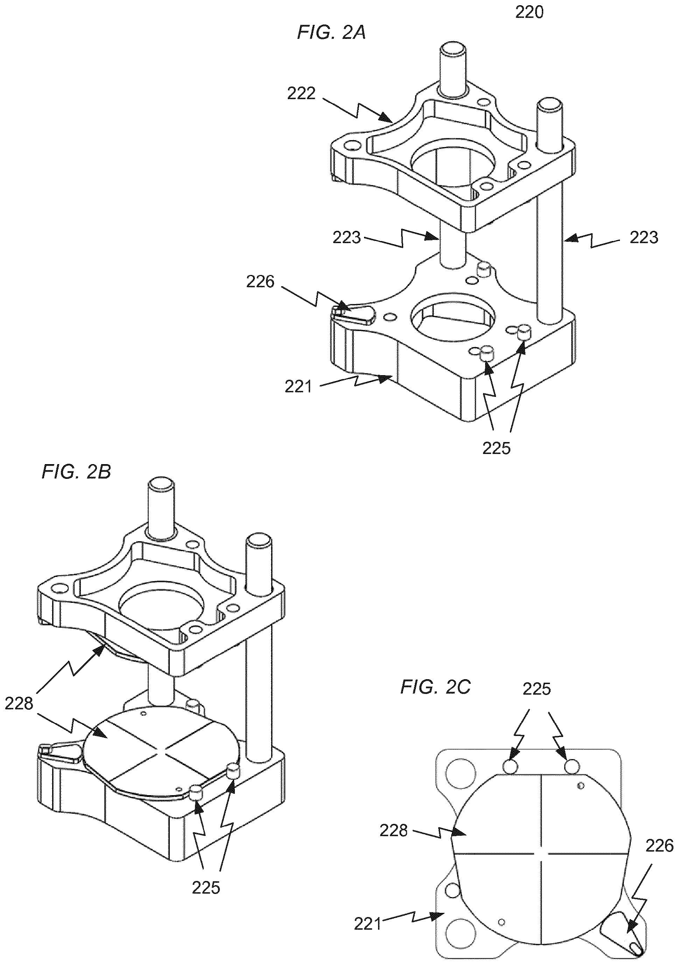

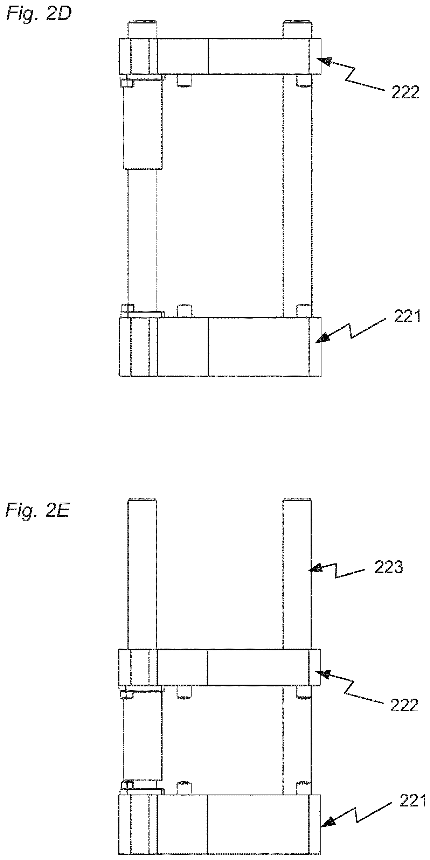

Fig. 2 shows an embodiment of an occlusion setup fixture.
Fig. 2A shows an occlusion setup fixture 220 with a first platform 221 and a second platform 222 which both are configured for engaging the linear guiding structure defined by two elongated posts 223. In the illustrated embodiment the elongated posts are secured in the first platform 221 while the second platform 222 is capable of sliding along the posts such that a linear translation along a linear axis is realized. The platforms each has a number of alignment structures 225 for arranging an interface plate in a predetermined and known arrangement on each platform and a means 226 for holding the interface plate in place during movement of the platforms (only the structures of the first platform can be seen in the figure).

In Fig. 2B interface plates 228 are arranged at the platforms (only the edge of the interface plate on the platform attached to the upper platform is visible). The interface plates are shaped such that they can only be held in the predetermined arrangement by the alignment structures 225.

Fig. 2C shows an interface 228 plate arranged on a platform 221 as seen along the linear axis of the occlusion setup fixture. A physical teeth model can be arranged in the interface plate using a range of suitable materials known to the skilled person.

Figs. 2D and 2E show the translation of the second platform along the guiding structure of the occlusion setup fixture. In Fig. 2D the platforms 221, 222 are arranged with their maximum distance to provide space for an operator to arrange interface plates and teeth models in the occlusion setup fixture. In Fig. 2E one platform 222 has been moved towards the other 221 along the guiding structure 223 such that the second interface plate can contact a second teeth model arranged in occlusion with a first teeth model arranged on an interface plate attached the other platform 221.

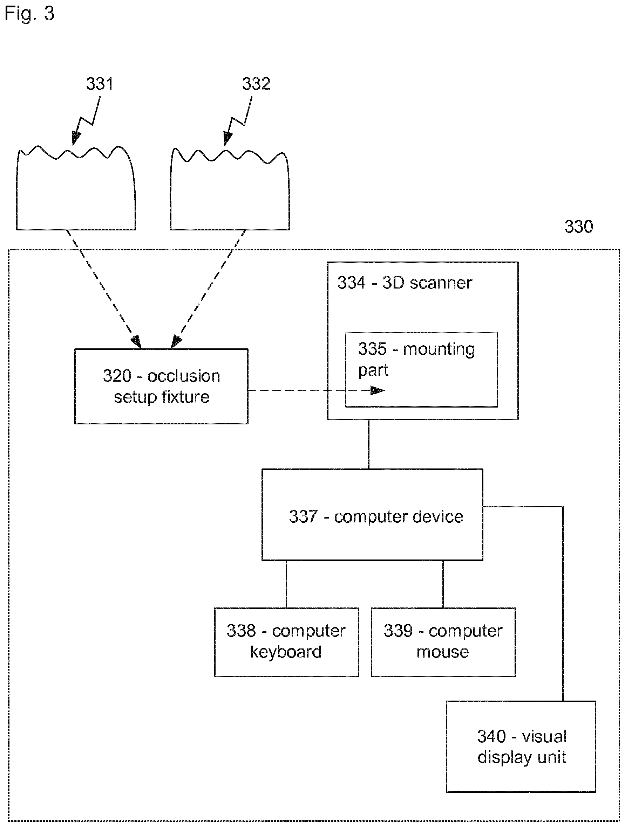

Fig. 3 shows a schematic of a system according to an embodiment. The system 330 comprises an occlusion setup fixture 320 according to any one of the embodiments, such as the fixture comprising a linear guiding structure defining a linear axis illustrated in Fig. 2. In the occlusion setup fixture a first and a second teeth model 331, 332 are arranged according to their occlusion. The physical teeth models are attached to interface plates configured for mating with platforms of the occlusion setup fixture and transferred to a mounting part 335 of a 3D scanner 334, where the mounting part is configured to provide that the interface plates are arranged in a known and predetermined orientation within the 3D scanner coordinate system. The digital 3D representations of the teeth models recorded by the 3D scanner are transferred to a computer device 337 comprising a computer readable medium and a processor. The system further comprises a visual display unit 340, a computer keyboard 338 and a computer mouse 339 for entering data and activating virtual buttons visualized on the visual display unit 340. The visual display unit 340 can be a computer screen. The computer device 337 is capable of receiving a digital 3D representation of the patient's set of teeth from the 3D scanner 334 or capable of receiving scan data from such a 3D scanner and forming a digital 3D representation of the patient's set of teeth based on such scan data. The received or formed digital 3D representation can be stored in the computer readable medium and provided to the processor. The processor is configured for executing computer implemented algorithms for virtually bringing the recorded digital 3D representations into occlusion by a virtual movement comprising a virtual translation along the linear axis of the occlusion setup fixture and for determining a digital transformation for arranging the digital 3D representations in a common coordinate system such that the digital 3D representations are arranged according to the patient's occlusion. The computer implemented algorithms can be configured for providing the various steps of the disclosed method.

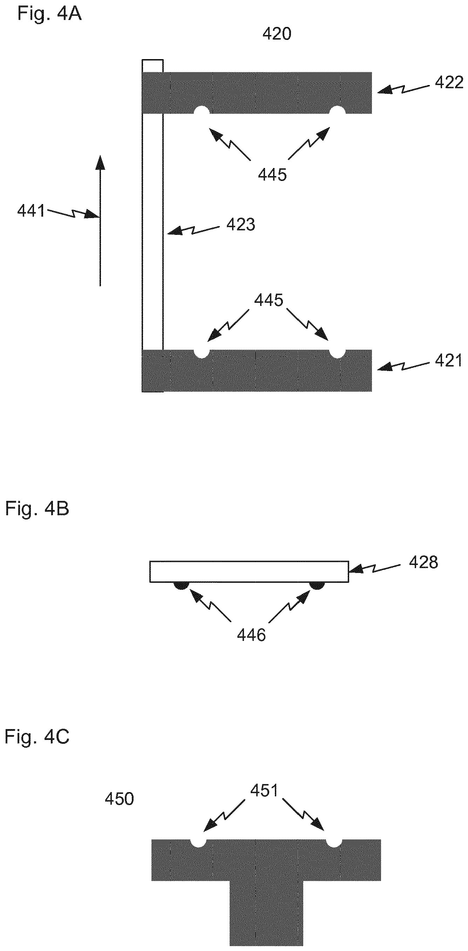

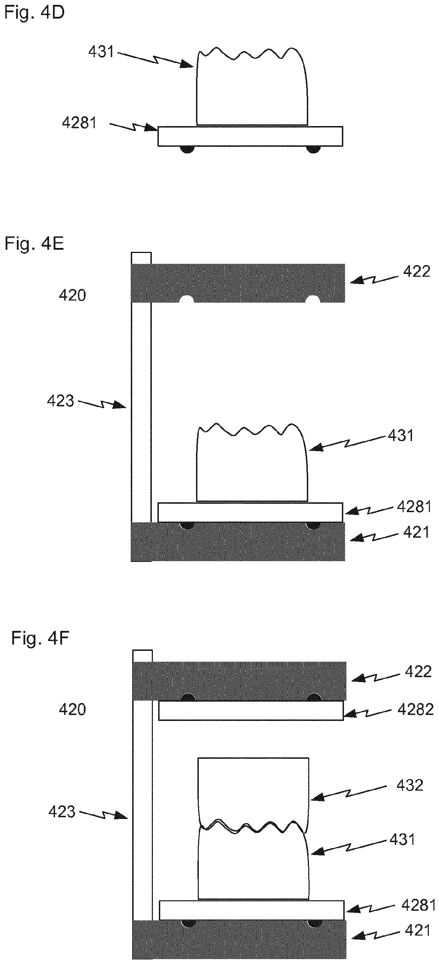

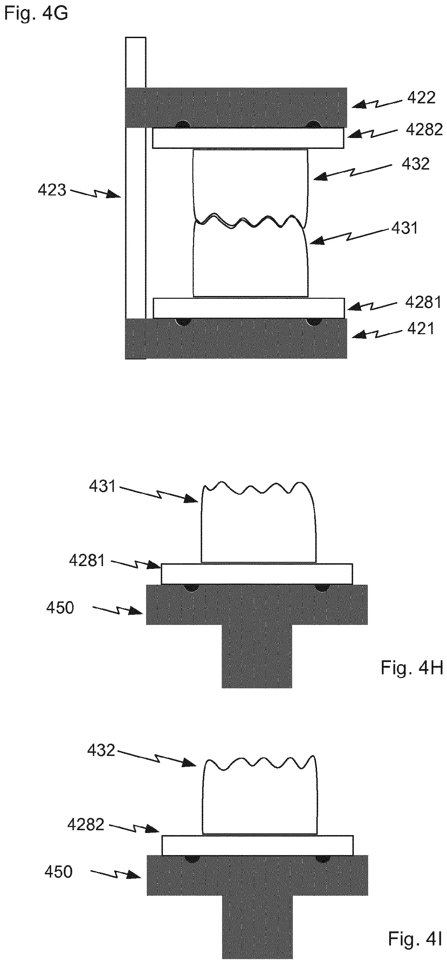

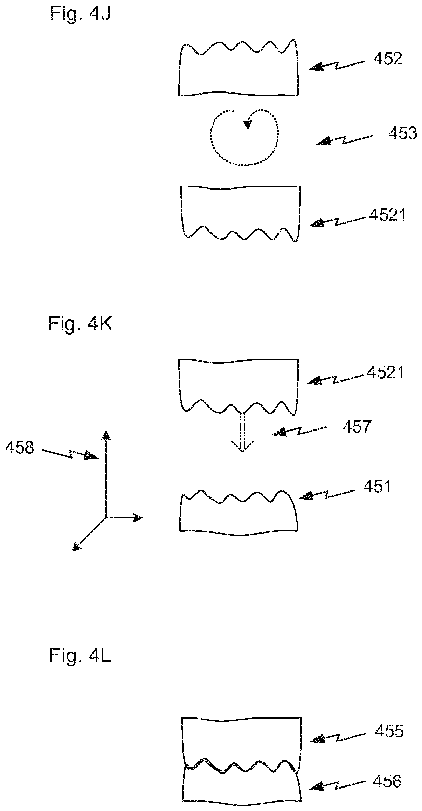

Fig. 4 illustrates a work-flow according to an embodiment. In this example, the first teeth model is of the teeth and soft tissue of the lower jaw, while the second teeth model is of the teeth and soft tissue of the upper jaw. The method is evidently not limited to this selection

Figs. 4A and 4B show schematics of an occlusion setup fixture 420 with platforms 421, 422 engaging a guiding structure 423 which defines a linear axis 441. The model interfaces of the fixture has interface plates 428 with magnetic balls 446 located in a pattern which matches the pattern of magnetic structures 445 in the platforms. When the interface plates are arranged at the platforms with the magnetic balls 446 mating with the structures 445 the interface plates are arranged according to a predetermined position and orientation relative to the platforms. An advantage of using magnetic units is that the interface plate can be attached relatively firmly at the platform while it is still possible to separate the two without using excessive force.

Fig. 4C shows a schematic of a fixed mounting part 450 of a 3D scanner with structures 451 for mating with the magnetic balls of the interface plate to provide that the interface plate is arranged according to a predetermined arrangement in the 3D scanner.

In Figs. 4D to 4G the teeth model 431 of the lower jaw is arranged at the first interface plate 4281 such that a first assembly is formed, and the first assembly is arranged at the first platform 421 in the occlusion setup fixture 420. The first interface plate 4281 engages with the first platform 421 in a predetermined orientation and position due to the magnetic alignment structures on the interface plate and platform.

The operator then places the teeth model 432 of the upper jaw in occlusion with the teeth model 431 of the lower jaw. This can be done based on a best fit of the teeth models and the experience of the operator. The second interface plate 4282 is arranged at the second platform 422 according to a predetermined arrangement dictated by the magnetic alignment structures of the second platform and interface plate.

The second interface plate 4282 is then brought into contact with the teeth model 432 of the upper jaw to form the second assembly while the teeth model 432 of the upper jaw is in occlusion with the teeth model of the lower jaw 431 as illustrated in Fig. 4G.

When the first and second assemblies are formed, they are removed from the occlusion setup fixture and arranged at the mounting part 450 of a 3D scanner as illustrated in Figs. 4H and 4I.

Digital 3D representations of the teeth models of the upper and lower teeth models are then recorded with the same arrangement of the mounting part in the 3D scanner during the two recordings. Due to the alignment structures on the interface plates and the mounting part, the first and second interface plates then also have the same arrangement in the 3D scanner during the recordings.

For ease of reading the phrase "upper digital 3D representation" is used in the following as short for the "digital 3D representation of the upper teeth model", and the phrase "lower digital 3D representation" is used instead of "digital 3D representation of the lower teeth model".

The mounting part and the interface plates are designed to provide that the position and orientation of the interface plate in the coordinate system of the 3D scanner is such that the normal of the interface plate is aligned with the z-axis of the 3D scanner coordinate system and the direction of the plate in the x-y plane of this coordinate system is according to a predetermined direction. However, due to manufacturing inaccuracies, such as inaccuracies in the arrangement of the magnetic alignment structures, the interface plate may be slightly tilted, rotated and offset from the planned arrangement such that its model engaging surface is misaligned with the coordinate system of the 3D scanner. One example of such misalignment is seen when the normal of the model engaging surface is not parallel to the z-axis of the coordinate system.

To compensate for this, an offset correcting transformation is applied to the upper and lower digital 3D representations.

The offset-correcting transformation is determined from a calibration scan, made e.g. once a week, where an interface plate is scanned without a teeth model arranged thereon. Known structures on the interface plate and/or the circumference of the interface plate are then identified in the recorded digital 3D representation and used for deriving the tilt, rotation and offset of the interface plate relative to the 3D scanner coordinate system. From this the offset-correcting transformation can be determined. Often the same offset-correcting transformation can be applied to the upper and lower digital 3D representations. The offset-corrected digital 3D representations are then arranged in the 3D scanner coordinate system in the manner which they would be if the interface plate was perfectly aligned with the 3D scanner coordinate system.

When arranged in the 3D scanner the upper and lower teeth models are arranged with the teeth facing the optical system of the 3D scanner. The digital 3D representations are hence recorded with the same orientation of the teeth models relative to the 3D scanner coordinate system. In order to provide that the upper digital 3D representation is rotated correctly relative to the lower digital 3D representation, a virtual rotation is applied to the upper digital 3D representation. The virtual rotation rotates the upper digital 3D representation 180 degrees around a rotation axis which is parallel to the patient's occlusal and medial planes or to the interface plate, such that the teeth in the rotated upper digital 3D representation faces downwards. This is illustrated in Fig. 4J where the virtual rotation 453 takes the upper digital 3D representation 452 and rotates it such that the teeth portion faces downwards after the rotation.

After the rotation, the digital 3D representations of the upper and lower teeth models are still overlapping. A virtual separation can thus be applied to the upper digital 3D representation where the movement of the digital 3D representation by the virtual separation is along the linear axis 441 of the occlusion setup fixture.

After the offset-correcting transformations, the virtual 180 degree rotation and the virtual separation, the upper digital 3D representation 452 and the lower digital 3D representation 451 are arranged with teeth surfaces facing each other and in the correct relative arrangement in the x-y plane of the 3D scanner coordinate system 458 as illustrated in Fig. 4K. The digital 3D representations are however separated in the direction of the z-axis.

In order to bring the digital 3D representations into occlusion, a virtual translation 457 according to the confined relative movement along the linear axis is applied to the upper digital 3D representation until the digital 3D representations collide. The digital 3D representations are then arranged according to the patient's occlusion in the 3D scanner coordinate system.

In principle the virtual separation and virtual translation can be replaced by a single virtual motion moving the rotated upper digital 3D representation along the linear axis until the upper and lower digital 3D representations only overlap in one point. This can provide the same translation of the upper digital 3D representation along the confined relative movement as what is obtained by the separating and connecting transformations.

In some cases a virtual occlusion optimization is also applied to the upper digital 3D representation to correct for e.g. a slight offset, tilt and rotation between the upper and lower teeth models when these are arranged according to the occlusion. This optimization can e.g. comprise a virtual tilting of the upper digital 3D representation in which parts of the upper digital 3D representation which still are at some distance from the corresponding parts of the lower digital 3D representation are pulled towards this and/or parts which are overlapping are pushed away. Further a rotation of the upper digital 3D representation around the z-axis of the 3D scanner system can be applied to correct for a slight error in the relative rotation of the upper and lower teeth models.

After the abovementioned transformations are applied to the upper and lower digital 3D representations the operator may choose to apply an inverse offset correcting transformation to both digital 3D representations to provide that the digital 3D representations describe the actual arrangement of the teeth models in the 3D scanner coordinate system.

The overall effect of the steps described above is that the recorded digital 3D representations has been brought into occlusion in the coordinate system of the 3D scanner.

A digital transformation for arranging the digital 3D representations according to the occlusion in the 3D scanner coordinate system can then be determined by e.g. matrix multiplication of the matrices describing the different steps, such that the effect of the digital transformation is to bring the recorded upper and lower digital 3D representations into occlusion.

Although some embodiments have been described and shown in detail, the invention is not restricted to them, but may also be embodied in other ways within the scope of the subject matter defined in the following claims. In particular, it is to be understood that other embodiments may be utilized and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

A claim may refer to any of the preceding claims, and "any" is understood to mean "any one or more" of the preceding claims.

It should be emphasized that the term "comprises/comprising" when used in this specification is taken to specify the presence of stated features, integers, steps or components but does not preclude the presence or addition of one or more other features, integers, steps, components or groups thereof.

The features of the method described above and in the following may be implemented in software and carried out on a data processing system or other processing means caused by the execution of computer-executable instructions. The instructions may be program code means loaded in a memory, such as a RAM, from a storage medium or from another computer via a computer network. Alternatively, the described features may be implemented by hardwired circuitry instead of software or in combination with software.

## Claims

1. A method for recording digital 3D representations of physical teeth models of a patient's jaws and for arranging the digital 3D representations in a common coordinate system such that the digital 3D representations are arranged according to the patient's occlusion, said method comprising:
- obtaining an occlusion setup fixture comprising a first and a second model interface each configured for supporting a teeth model and a guiding structure configured for providing a confined relative movement of the first and second model interfaces in the fixture;
- obtaining a first teeth model and attaching it to said first model interface such that a first assembly comprising the first teeth model and at least part of the first model interface is formed;
- obtaining a second teeth model and arranging it in occlusion with the first teeth model;
- attaching the second model interface to the second teeth model while the first and second teeth models are kept in occlusion such that a second assembly comprising the second teeth model and at least part of the second model interface is formed, where the second model interface and the second teeth model are brought into contact by the confined relative movement of the model interfaces;
- placing the first and second assemblies in a 3D scanner and recording a first and a second digital 3D representation of the first and second teeth models, respectively, where the relative arrangement of the model interface parts of the assemblies within the coordinate system of the 3D scanner is detected or predetermined;
- obtaining information describing the confined relative movement of the model interfaces in the occlusion setup fixture; and
- virtually bringing the recorded digital 3D representations into occlusion by a virtual movement, where the virtual movement comprises a virtual translation along a path according to the confined relative movement.

2. The method according to claim 1, wherein the confined relative movement comprises a translation along a linear axis of the fixture.

3. The method according to claim 1, wherein the fixture comprises an articulator and the confined relative movement of the model interfaces mimics the patient's bite.

4. The method according to any of claims 1, 2, or 3 wherein the virtual movement comprises a virtual 180 degree rotation of one of said digital 3D representations.

5. The method according to any one of claims 1 to 4, wherein the fixture comprises a first platform configured for engaging said guiding structure and the first model interface comprises a first interface plate configured for mating with the first platform in a predetermined orientation and position, and where the method comprises attaching the first teeth model and the first interface plate to the first platform such that the first assembly is attached to the first platform.

6. The method according to any one of claims 1 to 5, wherein the fixture comprises a second platform configured for engaging said guiding structure and the second model interface comprises a second interface plate configured for mating with the second platform in a predetermined orientation and position, and wherein the method comprises arranging the second interface plate at the second platform before attaching the second teeth model to the second interface plate.

7. The method according to any one of claims 1 to 6, wherein the method comprises performing a calibration scan to determine the position and orientation of the model engaging surface of the model interface in the coordinate system of the 3D scanner, and where the virtual movement comprises the rotation and/or movement required to compensate for a misalignment of the model engaging surface relative to the 3D scanner coordinate system.

8. The method according to any one of claims 1 to 7, wherein a collision detection between the first and second digital 3D representations is used to determine when to stop the virtual translation.

9. The method according to claim 8, wherein the method comprises an occlusion optimization where an optimized relative arrangement of the first and second digital 3D representations is detected and the virtual movement comprises a move to the detected optimized relative arrangement.

10. The method according to any one of claims 1 to 9, wherein the arrangement of the interface parts of the first and/or second assembly within the coordinate system of the 3D scanner is detected from structures of the model interface identified in the first and/or second digital 3D representations, respectively.

11. A scanner system, comprising means suitable for carrying out the method of claim 1, for recording digital 3D representations of physical teeth models of a patient's jaws and for arranging the digital 3D representations in a common coordinate system such that the digital 3D representations are arranged according to the patient's occlusion, said system comprising:
- an occlusion setup fixture comprising a first and a second model interface each configured for supporting a teeth model and a guiding structure configured for providing a confined relative movement of the first and second model interfaces in the fixture;
- a 3D scanner configured for recording digital 3D representations of the teeth models; and
- a data processing unit comprising a non-transitory computer readable medium on which is stored computer implemented algorithms for virtually bringing the recorded digital 3D representations into occlusion by a virtual movement, where the virtual movement comprises a translation along a path according to the confined relative movement.

12. The scanner system according to claim 11, wherein the guiding structure comprises one or more elongated posts defining a linear axis of the fixture, and where the first and second model interfaces are configured for directly or indirectly engaging said one or more elongated posts such that the relative movement of the first and second model interfaces is confined to a linear movement along the linear axis.

13. The scanner system according to claim 11 or 12, wherein the 3D scanner comprises a mounting part configured for mating with at least parts of the model interfaces in such a manner that the model interfaces are arranged according to a predetermined position and orientation in the 3D scanner.

14. The scanner system according to claim 11, 12 or 13, wherein at least one of said model interface has structures or a shape which can be identified in a digital 3D representation and used for determining the position and orientation of the model interface in the 3D scanner.

15. A method for recording digital 3D representations of physical teeth models of a patient's jaws and determining a digital transformation for arranging the digital 3D representations in a common coordinate system such that the digital 3D representations are arranged according to the patient's occlusion, said method comprising:
- bringing the recorded digital 3D representations into occlusion in a common coordinate system using the method according to one or more of claims 1 to 10; and
- determining the digital transformation from the virtual movements used to bring the digital 3D representations into occlusion.

## Patentansprüche

1. Verfahren zum Aufzeichnen von digitalen 3D-Darstellungen von physischen Zahnmodellen der Kiefer eines Patienten und zum Anordnen der digitalen 3D-Darstellungen in einem gemeinsamen Koordinatensystem, so dass die digitalen 3D-Darstellungen gemäß der Okklusion des Patienten angeordnet sind, wobei das Verfahren umfasst:
- Erhalten einer Okklusionseinspannvorrichtung, umfassend eine erste und eine zweite Modellschnittstelle, die jeweils ausgelegt sind zum Halten eines Zahnmodells und einer Führungsstruktur, die ausgelegt ist zum Bereitstellen einer begrenzten relativen Bewegung der ersten und zweiten Modellschnittstelle in der Spannvorrichtung;
- Erhalten eines ersten Zahnmodells und Anbringen desselben an der ersten Modellschnittstelle, so dass eine erste Baugruppe gebildet wird, die das erste Zahnmodell und mindestens einen Teil der ersten Modellschnittstelle umfasst;
- Erhalten eines zweiten Zahnmodells und Anordnen desselben in Okklusion mit dem ersten Zahnmodell;
- Anbringen der zweiten Modellschnittstelle an dem zweiten Zahnmodell, während das erste und das zweite Zahnmodell Okklusion gehalten werden, so dass eine zweite Baugruppe gebildet wird, die das zweite Zahnmodell und mindestens einen Teil der zweiten Modellschnittstelle umfasst, wobei die zweite Modellschnittstelle und das zweite Zahnmodell durch die begrenzte relative Bewegung der Modellschnittstellen in Berührung gebracht werden;
- Positionieren der ersten und der zweiten Baugruppe in einen 3D-Scanner und Aufzeichnen einer ersten und einer zweiten digitalen 3D-Darstellung des ersten und respektive des zweiten Zahnmodells, wobei die relative Anordnung der Modellschnittstellenteile der Baugruppen innerhalb des Koordinatensystems des 3D-Scanners erkannt wird oder vorbestimmt ist;
- Erhalten von Informationen, die die begrenzte relative Bewegung der Modellschnittstellen in der Okklusionseinspannvorrichtung beschreiben; und
- virtuelles Bringen in Okklusion der aufgezeichneten digitalen 3D-Darstellungen durch eine virtuelle Bewegung, wobei die virtuelle Bewegung eine virtuelle Translation entlang eines Wegs gemäß der begrenzten relativen Bewegung umfasst.

2. Verfahren nach Anspruch 1, wobei die begrenzte relative Bewegung eine Translation entlang einer linearen Achse der Spannvorrichtung umfasst.

3. Verfahren nach Anspruch 1, wobei die Spannvorrichtung einen Artikulator umfasst, und die begrenzte relative Bewegung der Modellschnittstellen den Biss des Patienten nachahmt.

4. Verfahren nach einem der Ansprüche 1, 2, oder 3, wobei die virtuelle Bewegung eine virtuelle Rotation der digitalen 3D-Darstellungen um 180 Grad umfasst.

5. Verfahren nach einem der Ansprüche 1 bis 4, wobei die Spannvorrichtung eine erste Plattform umfasst, die ausgelegt ist zum Eingreifen in die Führungsstruktur, und wobei die erste Modellschnittstelle eine erste Schnittstellenplatte umfasst, die ausgelegt ist, um in die erste Plattform in einer vorbestimmten Ausrichtung und Position einzupassen, und wobei das Verfahren das Anbringen des ersten Zahnmodell und der ersten Schnittstellenplatte an der ersten Plattform umfasst, so dass die erste Baugruppe an der ersten Plattform angebracht ist.

6. Verfahren nach einem der Ansprüche 1 bis 5, wobei die Spannvorrichtung eine zweite Plattform umfasst, die ausgelegt ist zum Eingreifen in die Führungsstruktur, und wobei die zweite Modellschnittstelle eine zweite Schnittstellenplatte umfasst, die ausgelegt ist, um in die zweite Plattform in einer vorbestimmten Ausrichtung und Position einzupassen, und wobei das Verfahren das Anordnen der zweiten Schnittstellenplatte an der zweiten Plattform vor Anbringen des zweiten Zahnmodell an der zweiten Schnittstellenplatte umfasst.

7. Verfahren nach einem der Ansprüche 1 bis 6, wobei das Verfahren das Durchführen eines Kalibrierscans umfasst, um die Position und die Ausrichtung der in das Modell eingreifenden Oberfläche der Modellschnittstelle im Koordinatensystem des 3D-Scanners zu bestimmen, und wobei die virtuelle Bewegung die erforderliche Rotation und/oder Bewegung umfasst, um eine Fehljustierung der in das Modell eingreifenden Oberfläche im Verhältnis zum Koordinatensystem des 3D-Scanners auszugleichen.

8. Verfahren nach einem der Ansprüche 1 bis 7, wobei eine Kollisionserkennung zwischen der ersten und der zweiten digitalen 3D-Darstellung verwendet wird, um zu bestimmen, wann die virtuelle Translation angehalten werden muss.

9. Verfahren nach Anspruch 8, wobei das Verfahren eine Okklusionsoptimierung umfasst, wobei eine optimierte relative Anordnung der ersten und der zweiten digitalen 3D-Darstellung erkannt wird, und die virtuelle Bewegung eine Verschiebung zur erkannten optimierten relativen Anordnung umfasst.

10. Verfahren nach einem der Ansprüche 1 bis 9, wobei die Anordnung der Schnittstellenteile der ersten und/oder zweiten Baugruppe innerhalb des Koordinatensystems des 3D-Scanners aus Strukturen der Modellschnittstelle erkannt wird, die in der ersten und/oder respektive zweiten digitalen 3D-Darstellung identifiziert wurden.

11. Scannersystem, umfassend Mittel, die geeignet sind zum Ausführen des Verfahrens nach Anspruch 1 zum Aufzeichnen von digitalen 3D-Darstellungen von physischen Zahnmodellen der Kiefer eines Patienten und zum Anordnen der digitalen 3D-Darstellungen in einem gemeinsamen Koordinatensystem, so dass die digitalen 3D-Darstellungen gemäß der Okklusion des Patienten angeordnet sind, wobei das System umfasst:
- eine Okklusionseinspannvorrichtung, umfassend eine erste und eine zweite Modellschnittstelle, die jeweils ausgelegt sind zum Halten eines Zahnmodells und einer Führungsstruktur, die ausgelegt ist zum Bereitstellen einer begrenzten relativen Bewegung der ersten und zweiten Modellschnittstelle in der Spannvorrichtung;
- einen 3D-Scanner, der ausgelegt ist zum Aufzeichnen von digitalen 3D-Darstellungen der Zahnmodelle; und
- eine Datenverarbeitungseinheit, umfassend ein nicht flüchtiges, computerlesbares Medium, auf dem computerimplementierte Algorithmen gespeichert sind zum virtuellen Bringen in Okklusion der aufgezeichneten digitalen 3D-Darstellungen durch eine virtuelle Bewegung, wobei die virtuelle Bewegung eine Translation entlang eines Wegs gemäß der begrenzten relativen Bewegung umfasst.

12. Scannersystem nach Anspruch 11, wobei die Führungsstruktur eine oder mehrere längliche Stützen umfasst, die eine lineare Achse der Spannvorrichtung definieren, und wobei das erste und das zweite Modellschnittstell ausgelegt sind für direktes oder indirektes Eingreifen der einen oder der mehreren länglichen Stützen, so dass die relative Bewegung der ersten und der zweiten Modellschnittstelle auf eine lineare Bewegung entlang der linearen Achse begrenzt ist.

13. Scannersystem nach Anspruch 11 oder 12, wobei der 3D-Scanner einen Montageteil umfasst, der ausgelegt ist, um in mindestens Teile der Modellschnittstellen so einzupassen, dass die Modellschnittstellen gemäß einer vorbestimmten Position und Ausrichtung im 3D-Scanner angeordnet sind.

14. Scannersystem nach Anspruch 11, 12 oder 13, wobei mindestens eine der Modellschnittstellen Strukturen oder eine Form aufweist, die in einer digitalen 3D-Darstellung identifiziert und zum Bestimmen der Position und Ausrichtung der Modellschnittstelle im 3D-Scanner verwendet werden können.

15. Verfahren zum Aufzeichnen von digitalen 3D-Darstellungen von physischen Zahnmodellen der Kiefer eines Patienten und Bestimmen einer digitalen Umgestaltung zum Anordnen der digitalen 3D-Darstellungen in einem gemeinsamen Koordinatensystem, so dass die digitalen 3D-Darstellungen gemäß der Okklusion des Patienten angeordnet sind, wobei das Verfahren umfasst:
- Bringen in Okklusion der aufgezeichneten digitalen 3D-Darstellungen in einem gemeinsamen Koordinatensystem unter Verwendung des Verfahrens nach einem oder mehreren der Ansprüche 1 bis 10; und
- Bestimmen der digitalen Umgestaltung aus den virtuellen Bewegungen, die verwendet wurden, um die digitalen 3D-Darstellungen in Okklusion zu bringen.

## Revendications

1. Procédé pour enregistrer des représentations 3D numériques de modèles de dent physiques des mâchoires d'un patient et pour agencer les représentations 3D numériques dans un système de coordonnées commun de telle sorte que les représentations 3D numériques sont agencées en fonction de l'occlusion du patient, ledit procédé comprenant :
- l'obtention d'un appareil de réparation des occlusions comprenant une première et une seconde interface de modèle configurées chacune pour supporter un modèle de dent et une structure de guidage configurée pour fournir un mouvement relatif confiné des première et seconde interfaces de modèle dans l'appareil ;
- l'obtention d'un premier modèle de dent et sa fixation à ladite première interface de modèle de telle sorte qu'un premier ensemble comprenant le premier modèle de dent et au moins une partie de la première interface de modèle est formé ;
- l'obtention d'un deuxième modèle de dent et son agencement en occlusion avec le premier modèle de dent ;
- la fixation de la seconde interface de modèle au second modèle de dent alors que les premier et second modèles de dent sont maintenus en occlusion de telle sorte qu'un second ensemble comprenant le second modèle de dent et au moins une partie de la seconde interface de modèle est formé, où la seconde interface de modèle et le second modèle de dent sont amenés en contact par le mouvement relatif confiné des interfaces de modèle ;
- la mise en place des premier et second ensembles dans un dispositif de balayage 3D et l'enregistrement d'une première et d'une seconde représentation 3D des premier et second modèles de dent, respectivement, où l'agencement relatif des parties d'interface de modèle des ensembles dans le système de coordonnées du dispositif de balayage 3D est détecté ou prédéterminé ;
- l'obtention d'informations décrivant le mouvement relatif confiné des interfaces de modèle dans l'appareil de réparation des occlusions ; et
- le fait d'amener virtuellement les représentations 3D numériques en occlusion par un déplacement virtuel, où le mouvement virtuel comprend une translation virtuelle le long d'un chemin en fonction du mouvement relatif confiné.

2. Procédé selon la revendication 1, dans lequel le mouvement relatif confiné comprend une translation le long d'un axe linéaire de l'appareil.

3. Procédé selon la revendication 1, dans lequel l'appareil comprend un articulateur et le mouvement relatif confiné des interfaces de modèle imite la morsure du patient.

4. Procédé selon l'une quelconque des revendications 1, 2 ou 3 dans lequel le mouvement virtuel comprend une rotation virtuelle de 180 degrés de l'une desdites représentations 3D numériques.

5. Procédé selon l'une quelconque des revendications 1 à 4, dans lequel l'appareil comprend une première plateforme configurée pour entrer en prise avec ladite structure de guidage et la première interface de modèle comprend une première plaque d'interface configurée pour s'accoupler avec la première plateforme dans des orientation et position prédéterminées, et où le procédé comprend la fixation du premier modèle de dent et de la première plaque d'interface à la première plateforme de telle sorte que le premier ensemble est fixé à la première plateforme.

6. Procédé selon l'une quelconque des revendications 1 à 5, dans lequel l'appareil comprend une seconde plateforme configurée pour entrer en prise avec ladite structure de guidage et la seconde interface de modèle comprend une seconde plaque d'interface configurée pour s'accoupler avec la seconde plateforme dans des orientation et position prédéterminées, et où le procédé comprend l'agencement de la seconde plaque d'interface au niveau de la seconde plateforme avant la fixation du second modèle de dent à la seconde plaque d'interface.

7. Procédé selon l'une quelconque des revendications 1 à 6, dans lequel le procédé comprend la réalisation d'un balayage d'étalonnage pour déterminer la position et l'orientation de la surface de mise en prise de modèle de l'interface de modèle dans le système de coordonnées du dispositif de balayage 3D, et où le mouvement virtuel comprend la rotation et/ou le mouvement nécessaires pour compenser un mauvais alignement de la surface de mise en prise de modèle par rapport au système de coordonnées de dispositif de balayage 3D.

8. Procédé selon l'une quelconque des revendications 1 à 7, dans lequel une détection de collision entre les première et seconde représentations 3D numériques est utilisée pour déterminer le moment où il faut arrêter la translation virtuelle.

9. Procédé selon la revendication 8, dans lequel le procédé comprend une optimisation d'occlusion où un agencement relatif optimisé des première et seconde représentations 3D numériques est détecté et le mouvement virtuel comprend un mouvement vers l'agencement relatif optimisé détecté.

10. Procédé selon l'une quelconque des revendications 1 à 9, dans lequel l'agencement des parties d'interface du premier et/ou second ensemble dans le système de coordonnées du dispositif de balayage 3D est détecté à partir de structures de l'interface de modèle identifié dans les première et/ou seconde représentations 3D numériques, respectivement.

11. Système à dispositif de balayage, comprenant un moyen approprié pour réaliser le procédé selon la revendication 1, pour enregistrer des représentations 3D numériques de modèles de dent physiques des mâchoires d'un patient et pour agencer les représentations 3D numériques dans un système de coordonnées commun de telle sorte que les représentations 3D numériques sont agencées en fonction de l'occlusion du patient, ledit système comprenant :
- un appareil de réparation des occlusions comprenant une première et une seconde interface de modèle configurées chacune pour supporter un modèle de dent et une structure de guidage configurée pour fournir un mouvement relatif confiné des première et seconde interfaces de modèle dans l'appareil ;
- un dispositif de balayage 3D configuré pour enregistrer des représentations 3D numériques des modèles de dent ; et
- une unité de traitement de données comprenant un support lisible par ordinateur non transitoire sur lequel sont stockés des algorithmes implémentés par ordinateur pour amener virtuellement les représentations 3D numériques enregistrées en occlusion par un mouvement virtuel, où le mouvement virtuel comprend une translation le long d'un chemin en fonction du mouvement relatif confiné.

12. Système à dispositif de balayage selon la revendication 11, dans lequel la structure de guidage comprend un ou plusieurs montants allongés définissant un axe linéaire de l'appareil, et où les première et seconde interfaces de modèle sont configurées pour entrer en prise directe ou indirecte avec lesdits un ou plusieurs montants allongés de telle sorte que le mouvement relatif des première et seconde interfaces de modèle est confiné à un mouvement linéaire le long de l'axe linéaire.

13. Système à dispositif de balayage selon la revendication 11 ou 12, dans lequel le dispositif de balayage 3D comprend une partie de montage configurée pour s'accoupler avec au moins des parties des interfaces de modèle d'une manière telle que les interfaces de modèle sont agencées en fonction d'une position et d'une orientation prédéterminées dans le dispositif de balayage 3D.

14. Système à dispositif de balayage selon la revendication 11, 12 ou 13, dans lequel au moins une desdites interfaces de modèle a des structures ou une forme qui peuvent être identifiées dans une représentation 3D numérique et utilisées pour déterminer la position et l'orientation de l'interface de modèle dans le dispositif de balayage 3D.

15. Procédé pour enregistrer des représentations 3D numériques de modèles de dent physiques des mâchoires d'un patient et déterminer une transformation numérique pour agencer les représentations 3D numériques dans un système de coordonnées commun de telle sorte que les représentations 3D numériques sont agencées en fonction de l'occlusion du patient, ledit procédé comprenant :
- le fait d'amener les représentations 3D numériques enregistrées en occlusion dans un système de coordonnées commun en utilisant le procédé selon une ou plusieurs des revendications 1 à 10 ; et
- la détermination de la transformation numérique à partir des mouvements virtuels utilisés pour amener les représentations 3D numériques en occlusion.
